# EUROPEAN PATENT SPECIFICATION

(11) **EP 1 073 702 B1**
(45) Date of publication and mention of the grant of the patent: **16.10.2002**
(21) Application number: 99923423.0
(22) Date of filing: 16.04.1999
(51) Int. Cl.: C11B 9/00, C07C 35/08

(54) **PERFUMES COMPRISING 4-ISOBUTYLCYCLOHEXANOLS**
4-ISOBUTYLCYCLOHEXANOL ENTHALTENDE RIECHSTOFFE
PARFUMS COMPRENANT DES 4-ISOBUTYLCYCLOHEYANOLS

(30) Priority: 24.04.1998 EP 98303219
(43) Date of publication of application: 07.02.2001
(73) Proprietor: QUEST INTERNATIONAL B.V., 1411 GP Naarden (NL)
(72) Inventor: YARWOOD, Kim Joyce, Kent CT20 3PF (GB); NEWMAN, Christopher Paul, Kent CT4 7BS (GB)
(74) Representative: Matthews, Heather Clare
(86) International application number: EP9902591
(87) International publication number: WO99055811

(56) References cited:
- EP-A- 0 005 196
- WO-A-98/47842
- US-A- 4 400 545
- US-A- 5 543 398

## Description

The invention relates to 4-isobutyl-cyclohexan-1-ols, to their use as fragrance materials and to perfumes and perfumed products comprising these alcohols.

In the perfumery art there is a continuous interest in new fragrance materials, on the one hand caused by a continuous need for compounds with a new odour character and on the other hand caused by the fact that many of the long known materials have come under scrutiny lately because of certain undesirable properties. Thus, some well known fragrance materials have been criticized because of their potential instability in some applications. Some of these are well known floral fragrances, such as α-hexylcinnamaldehyde, 2-methyl-3-(p-tert-butylphenyl)propanal, 2-methyl-3-(p-isopropylphenyl)propanal, 3-(p-tert-butylphenyl)propanal and hydroxycitronellal.

Some alkyl substituted cyclohexanols are long known in perfumery. Thus, S. Arctander, Perfume and Flavor Chemicals mentions 4-isopropyl-, 4-tert.butyl-, 4-tert.amyl-, 4-heptyl- and 2-methyl-4-tert.amyl-cyclohexanol (monograph no's 2692, 165, 433, 1531 and 1749). The odour character of all five compounds is overridingly woody with camphoraceous, piney, vetiver, cedar notes and also often dry with tarry or leathery odour notes. 2-Tert.butylcyclohexanol (monogr.no. 432) is also described as dry woody, camphoraceous and tarry.

4-Isoamylcyclohexanols, particularly di-, tri- and tetra-methyl substituted ones are disclosed in US 4,400,545 as fragrance materials with balsamic, woody, sweet, rooty, musty, earthy and leathery odours.

4-(C8-Alkyl)cyclohexanols are disclosed in EP0 005 196 as having an odour resembling that of 4-tert.butyl-cyclohexanol, but less strong. The most important compound is 4-(2,2,3,3-tetramethylbutyl)cyclohexanol.

In JP 02131405 (Hasegawa Koryo Co. Ltd) 2-, 3- and 4-alkylcycloalkanols and esters thereof are described as cockroach repellents. In passing it is stated that these compounds are used as fragrances, but for the alcohols this is not further substantiated than by referring to the Arctander monographs cited above, and no indication of odour character is given in this patent application.

In EP0 053 979 alkyl-substituted 3(2-alkenyl)-cyclopentanols are broadly disclosed with a wide variety of odour notes of the floral, fruity and woody type reminiscent of rose, muguet, bergamot, cucumber, hay-like, dry-woody, cedar or vetiver. The specific compounds shown are all unsubstituted 3(2-alkenyl)cyclopentanols and 1,2,2-trimethyl-3(2-alkenyl) cyclopentanols.

In US 4,277,618 1-methyl-2-C4-cyclohexanols (C4 = sec.butyl or tert.butyl) are disclosed as intermediates in the synthesis of the corresponding acetates. Only the latter are said to be fragrance materials.

In US 3,769,330 1-ethyl-2-C4-cyclohexanols (C4 = n.butyl, isobutyl, sec.butyl or tert.butyl) are disclosed as fragrance materials with a woody odour.

It has now been found that 4-isobutylcyclohexanols of the formula I below are valuable fragrance materials with strong muguet odours, unlike the related 4-tert.butyl-, tert.amyl- and isoamylcyclohexanols which all have a predominantly woody and leathery odour. 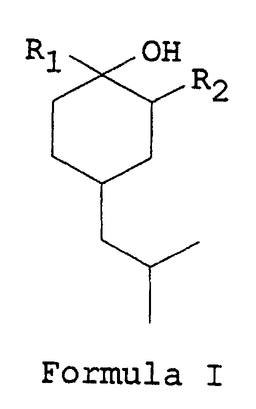 In Formula I R, represents hydrogen, or a methyl or ethyl group. Preferably R₁ is hydrogen or methyl. R₂ represents hydrogen or a methyl group. Preferably R₂ is hydrogen.

Thus, the invention provides perfumes and perfumed products comprising the alcohols according to Formula I. Furthermore, the invention provides a process for imparting a muguet odour note to perfumes and products to be perfumed. Finally, the invention provides novel fragrance materials according to Formula I above wherein R₁ is a methyl or ethyl group, as well as 2-methyl-4-isobutylcyclohexan-1-ol.

The alcohols according to the invention may be used as such in a wide variety of products, or they may be used as components of a perfume to contribute their muguet odour. This odour does not necessarily need to be the strongest or predominant odour note in the perfume or the final perfumed product, but it clearly contributes a muguet note to the overall odour of such perfume or perfumed product.

For the purposes of this invention a perfume is defined as a mixture of fragrance materials, if desired mixed with or dissoved in a suitable solvent or mixed with a solid substrate, which is used to impart a desired odour to the skin and/or any product for which an agreeable odour is indispensible or desirable. The alcohols according to the invention and the perfumes containing them are particularly suitable for use in e.g.: fabric washing powders, washing liquids, fabric softeners and other fabric care products; soaps, bath and shower gels, shampoos, hair conditioners and other personal cleansing products; cosmetic products such as creams, ointments, toilet waters, products for preshave or aftershave use, skin and other lotions, talcum powders, body deodorants and antiperspirants.

Other fragrance materials known in the art which can be advantageously combined with an alcohol according to the invention in a perfume are, for example, natural products such as extracts, essential oils, absolutes, resinoids, resins, concretes etc., but also synthetic materials such as hydrocarbons, alcohols, aldehydes, ketones, ethers, acids, esters, acetals, ketals, nitrites, etc., including saturated and unsaturated compounds, aliphatic, carbocyclic and heterocyclic compounds.

Such fragrance materials are mentioned, for example, in S. Arctander, Perfume and Flavor Chemicals (Montclair, N.J., 1969), in S. Arctander, Perfume and Flavor Materials of Natural Origin (Elizabeth, N.J., 1960) and in "Flavor and Fragrance Materials - 1991", Allured Publishing Co. Wheaton, I11. USA.

Examples of fragrance materials which can be used in combination with an alcohol according to the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, styrallyl acetate, benzyl benzoate, amyl salicylate, dimethylbenzyl-carbinol, trichloromethylphenylcarbinyl acetate, p-tert-butylcyclohexyl acetate, isononyl acetate, vetiveryl acetate, vetiverol, α hexylcinnamaldehyde, 2-methyl-3-(p-tert-butylphenyl)propanal, 2-methyl-3-(p-isopropylphenyl)propanal, 3-(p-tert-butylphenyl)-propanal, 2,4-dimethylcyclohex-3-enyl-carboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyl-tetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethyl-acetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks, tetralin musks, isochroman musks, macrocyclic ketones, macrolactone musks, ethylene brassylate.

Solvents which can be used for perfumes which contain alcohols according to the invention are, for example: ethanol, isopropanol, diethyleneglycol monoethyl ether, dipropylene glycol, diethyl phthalate, triethyl citrate, isopropyl myristate, etc.

The compounds according to the invention may be prepared according to procedures known in the art, particularly starting from 4-isobutylphenol and following the procedure described for the corresponding 4-isoamyl-cyclohexanols in US 4,400,545.

The alcohols according to the invention are generally obtained as mixtures of cis and trans isomers (OH and isobutyl on the same side or opposite sides of the ring respectively), the cis/trans ratio being dependent on the synthesis procedure. Generally the odours of both isomers are different and the isomers may be separated by procedures known in the art such as column chromatography, fractional distillation and gas chromatography. The isomers may be used separately as fragrance materials or the isomer mixtures obtained from the synthetic procedure may be used as such, depending on which particular odour character or mixture of odour characters is preferred for a particular application. Also, the alcohols according to the invention exist is various stereoisomeric forms. They are obtained by the synthetic procedures described above as racemic mixtures, which may be separated into the various stereo isomers by procedures known in the art, particularly by gas chromatography using chiral columns. Therefore, the invention provides the alcohols as cis/trans and stereo-isomeric mixtures as well as the various cis and trans and stereoisomers separately and includes the use of these separate isomers as fragrance materials.

The quantities in which an alcohol according to the invention can be used in perfumes or in products to be perfumed may vary within wide limits and depend, inter alia, on the nature of the product, on the nature and the quantity of the other components of the perfume in which the compound is used and on the olfactive effect desired. It is therefore only possible to specify wide limits, which, however, provide sufficient information for the specialist in the art to be able to use the alcohols according to the invention for his specific purpose. In perfumes an amount of 0.01% by weight or more of an alcohol according to the invention will generally have a clearly perceptible olfactive effect. Preferably the amount is 0.1% by weight or more, more preferably at least 1%. Generally it will be less than 80% by weight. The amount of alcohol according to the invention in a product will generally be at least 10 ppm by weight, preferably at least 100 ppm, more preferably at least 1000 ppm.
However, levels of up to about 20% by weight may be used in particular cases, depending on the product to be perfumed.

### EXAMPLE 1

### Preparation of 4-isobutylcyclohexan-1-ol

(1) To solid aluminium chloride (1.25mol) in a 1 litre three-necked round bottom flask was carefully added nitrobenzene (500mls). An exotherm was noticed during this addition. The resulting solution was cooled to 10°C using an ice bath. To this was then added isobutyryl chloride (0.52mol) and phenol (0.50mol, in nitrobenzene) via separate dropping funnels whilst ensuring similar rates of addition. A slight exotherm was noted (10-20°C) during the course of the addition. The reaction was stirred for a further 5 hours at room temperature and then left overnight. The reaction mixture was carefully poured into 1 litre of saturated aqueous sodium hydroxide solution. More sodium hydroxide was added to bring the pH to 10. This basic solution was then extracted with diethyl ether (2x500ml) and the organic fractions were discarded. The aqueous solution was acidified with sulphuric acid to pH1 and then extracted with diethyl ether (2x500ml). The organic fractions were combined and washed with water (3x500ml), dried with anhydrous magnesium sulphate, and then evaporated *in vacuo.* to yield a viscous residue (crude yield was 91% which was 96% pure by gas chromatography analysis). The residue was purified by distillation and/or recrystallisation (from diethyl ether/pentane) to yield colourless crystals of 1-(4-hydroxyphenyl)-2-methyl-1-propanone.
   ¹³C NMR (CDCl₃):206.0, 161.7, 131.5, 128.4, 116.0, 3b.3, 19.6.
   ¹H NMR (CDCl₃): 8.5 (1H), 8.0 (2H), 7.0 (2H), 3.6 (1H), 1.3 (6H).
(2) To 2-butanol (60ml) containing a suspension of 5% Rh/C catalyst (1g) was added 1-(4-hydroxyphenyl)-2-methyl-1-propanone (17g) produced in (1) above. The resulting suspension was placed under a total hydrogen pressure of 70bar and heated at 70°C whilst being followed by gas chromatography. Once complete the catalysts was removed by filtration through celite, and the solvent was evaporated *in vacuo.* The length of reaction time can be varied to yield almost entirely 4-isobutylcyclohexan-1-ol or a mix of this along with the corresponding ketone (4-isobutylcyclohexan-1-one). Any ketone contaminant was removed from the product mixture by treating the product mixture with excess lithium aluminium hydride in diethyl ether followed by a standard work up. The resulting crude alcohol was purified by distillation to give a colourless liquid of 4-isobutylcyclohexan-1-ol.
   ¹³C NMR (CDCl₃): (2 isomers) 71.2, 67.2, 46.2, 45.4, 35.6, 34.1, 33.5, 32.2, 31.4, 27.2, 25.0, 24.7, 22.9, 22.8.
   ¹H NMR (CDCl₃): 3.9 (1H), 3.5(1H), 1.9 (1H), 1.8(1H), 1.7-0.9 (10H), 1.7 (6H).

### Perfume Composition

A perfume composition of the floral type was prepared according to the following recipe:

| | |
|---|---|
| α-Terpineol | 12 parts |
| Jasmine absolue 50% | 4 parts |
| Heliotropin, 20% in DPG | 2 parts |
| Traseolide * | 1 part |
| Peru Balsam | 1 part |
| 4-isobutylcyclohexan-1-ol | 2 parts. |

| | |
|---|---|
| * Indane musk marketed by Quest International, Ashford Kent, U.K. | |

The addition of the alcohol according to the invention enhances the muguet character of the composition and diminishes the harsh notes of terpineol.

## Claims

1. A perfume comprising fragrance materials known in the art and at least one alcohol according to Formula I: 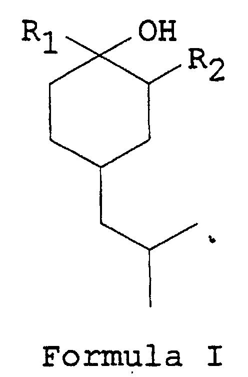 wherein R₁ represents hydrogen or a methyl or ethyl group and R₂ represents hydrogen or a methyl group.

2. A perfume according to claim 1 wherein R₁ represents hydrogen or a methyl group.

3. A perfume according to claims 1 or 2 wherein R₂ represents hydrogen.

4. A perfume according to claims 1-3 wherein the alcohol is present in an amount of at least 0.01% by weight.

5. A perfume composition according to claim 1 wherein the alcohol is chosen from 4-isobutylcyclohexan-1-ol and 2-methyl-4-isobutylcyclohexan-1-ol.

6. A process for imparting a muguet odour note to products to be perfumed, comprising the step of incorporating in the product a perfume according to any one of claims 1-5.

7. A process according to claim 6 wherein the product to be perfumed is chosen from: fabric washing powders, washing liquids, fabric softeners and other fabric care products; soaps, bath and shower gels, shampoos, hair conditioners and other personal cleansing products; cosmetic products such as creams, ointments, toilet waters, products for preshave or aftershave use, skin and other lotions, talcum powders, body deodorants and antiperspirants.

8. 4-lsobutylcyclohexanols according to formula I: 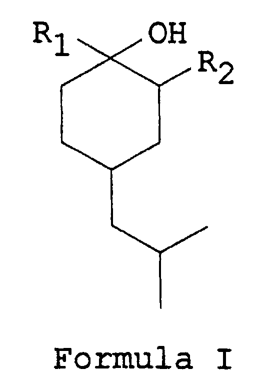 wherein R₁ represents a methyl or ethyl group and R₂ represents hydrogen or a methyl group.

9. 4-Isobutylcyclohexanols according to claim 8 wherein R₁ is a methyl group.

10. 2-methyl-4-isobutylcyclohexan-1-ol.

## Patentansprüche

1. Riechstoff, umfassend im Stand der Technik bekannte Duftmaterialien und mindestens einen Alkohol nach der Formel I: 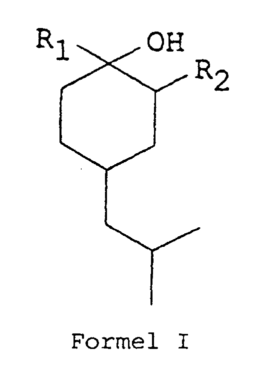 worin R₁ Wasserstoff oder eine Methyl- oder Ethylgruppe darstellt und R₂ Wasserstoff oder eine Methylgruppe darstellt.

2. Riechstoff nach Anspruch 1, worin R₁ Wasserstoff oder eine Methylgruppe darstellt.

3. Riechstoff nach Anspruch 1 oder 2, worin R₂ Wasserstoff darstellt.

4. Riechstoff nach den Ansprüchen 1 bis 3, worin der Alkohol in einer Menge von mindestens 0,01 Gew.% vorliegt.

5. Riechstoffzusammensetzung nach Anspruch 1, worin der Alkohol ausgewählt ist aus 4-Isobutylcyclohexan-1-ol und 2-Methyl-4-isobutylcyclohexan-1-ol.

6. Verfahren um zu parfümierenden Produkten eine Maiglöckchengeruchsnote zu verleihen, umfassend den Schritt der Einbeziehung eines Parfüms nach irgendeinem der Ansprüche 1 bis 5 in das Produkt.

7. Verfahren nach Anspruch 6, worin das zu parfümierende Produkt ausgewählt wird aus: Gewebewaschpulvern, Waschflüssigkeiten, Gewebweichmachern und anderen Gewebepflegeprodukten; Seifen, Bad- und Duschgelen, Shampoos, Haarkonditionierungsmitteln und anderen persönlichen Reinigungsprodukten, Kosmetikprodukten, wie Cremen, Salben, Toilettenwassern, Produkten zur Verwendung vor oder nach der Rasur, Haut- und anderen Lotionen, Talkumpuder, Körperdeoderantien und Antitranspirantien.

8. 4-Isobutylcyclohexanole nach der Formel I: 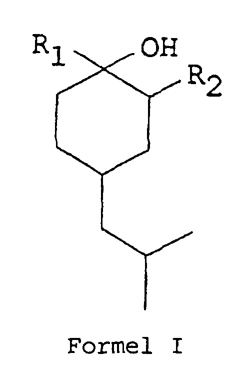 worin R₁ eine Methyl- oder Ethylgruppe darstellt und R₂ Wasserstoff oder eine Methylgruppe darstellt.

9. 4-Isobutylcyclohexanole nach Anspruch 8, worin R₁ eine Methylgruppe darstellt.

10. 2-Methyl-4-Isobutylcyclohexan-1-ol.

## Revendications

1. Parfum comprenant des matériaux de fragrance connus dans l'art et au moins un alcool selon la Formule I : 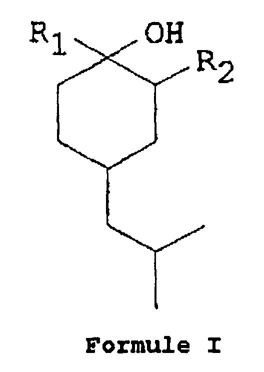 dans laquelle R₁ représente de l'hydrogène ou un groupe méthyle ou éthyle et R₂ représente de l'hydrogène ou un groupe méthyle.

2. Parfum selon la revendication 1, dans lequel R₁ représente de l'hydrogène ou un groupe méthyle.

3. Parfum selon les revendications 1 ou 2, dans lequel R₂ représente de l'hydrogène.

4. Parfum selon les revendications 1 à 3, dans lequel l'alcool est présent dans une quantité d'au moins 0,01% par poids.

5. Composition de parfum selon la revendication 1 dans laquelle l'alcool est choisi parmi le 4-isobutylcyclohexan-1-ol et 2-méthyl-4-isobutylcyclohexan-1-ol.

6. Procédé pour diffuser la note olfactive du muguet aux produits à parfumer, comprenant l'étape de l'incorporation d'un parfum dans le produit, selon l'une quelconque des revendications de 1 à 5.

7. Procédé selon la revendication 6, dans lequel le produit à parfumer est choisi parmi : les détergents industriels en poudre, les détergents liquides, les solvants industriels et autres produits d'entretien industriels ; les savons, les gels douche et les crèmes de bains, les shampooings, les revitalisants capillaires et autres produits pour l'hygiène personnelle ; les produits cosmétiques tels que les crèmes, onguents, les eaux de toilette, les produits de rasage ou les après-rasage, les lotions pour la peau et autres, les talcs en poudre, des déodorants corporels et les produits antisudoraux.

8. 4-lsobutylcyclohexanols selon la Formule 1 : 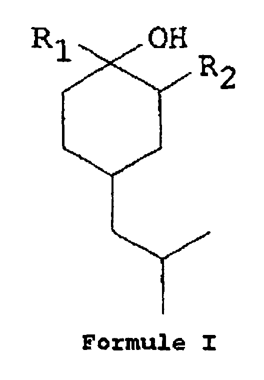 dans laquelle R₁ représente un groupe méthyle ou éthyle et R₂ représente de l'hydrogène ou un groupe méthyle.

9. 4-isobutylcyclohexanols selon la revendication 8 dans lesquels R₁ représente un groupe méthyle.

10. 2-méthyl-4-isobutylcyclohexan-1-ol.
